# EUROPÄISCHE PATENTSCHRIFT

(11) **EP 0 951 245 B1**
(45) Veröffentlichungstag und Bekanntmachung des Hinweises auf die Patenterteilung: **12.03.2003**
(21) Anmeldenummer: 96939801.5
(22) Anmeldetag: 12.12.1996
(51) Int. Cl.: A61B 17/70

(54) **VORRICHTUNG ZUR VERBINDUNG EINES LANGSTRAGERS MIT EINER PEDIKELSCHRAUBE**
DEVICE FOR CONNECTING A LONGITUDINAL SUPPORT TO A PEDICLE SCREW
DISPOSITIF POUR RELIER UN SUPPORT LONGITUDINAL AVEC UNE VIS PEDICULAIRE

(43) Veröffentlichungstag der Anmeldung: 27.10.1999
(73) Patentinhaber: SYNTHES AG Chur, 7002 Chur (CH)
(72) Erfinder: SCHLÄPFER, Fridolin, CH-8750 Glarus (CH)
(74) Vertreter: Lusuardi, Werther Giovanni, Dr.
(86) Internationale Anmeldenummer: CH9600437
(87) Internationale Veröffentlichungsnummer: WO98025534

(56) Entgegenhaltungen:
- EP-A- 0 330 881
- WO-A-94/00066
- DE-C- 19 509 332
- US-A- 5 549 608

## Beschreibung

Die Erfindung betrifft eine Vorrichtung gemäss dem Oberbegriff des Patentanspruchs 1.

Aus dem Stand der Technik sind bereits eine ganze Anzahl von Pedikelschrauben für die Wirbelsäulenfixation bekannt, welche den Vorteil besitzen, dass die einzelnen Pedikelschrauben unabhängig vom Längsträger implantiert werden können. Der Längsträger wird nachträglich entweder seitlich oder von oben in die Pedikelschraube eingeführt und fixiert. Eine solche Pedikelschraube ist beispielsweise aus der EP-B 0 330 881 SHERMAN bekannt.

Der Nachteil der bekannten Pedikelschrauben besteht in den zum Teil relativ komplizierten Verschlussmechanismen um den, in den offen ausgebildeten Pedikelschraubenkopf eingelegten Längsträger zu fixieren. Zudem lässt sich in den meisten Fällen der Kopf der Schraube nur bedingt zur Lage des Längsträgers ausrichten, was ein aufwendiges Zurechtbiegen des Längsträgers bedingt.

Aus der DE-C 195 09 332 ist ein Verankerungselement gemass dem Oberbegriff des Anspruchs 1 bekannt, bei welchem der kugelige Kopf einer Pedikelschraube in einem zusammenpressbaren Druckelement federnd eingeschnappt werden kann und dadurch eine gewisse Abwinkelung der Pedikelschraube ermöglicht.
Eine Verspannung des Druckelementes im Aufnahmekopf findet auf Grund eines schon in der PCT/CH92/00125 offenbarten Mechanismus statt, indem das kegelstumpfförmige Druckelement in einer komplementär kegeligen Bohrung gegen dessen verjüngendes Ende hin bewegt und dadurch verklemmt wird. Mit dem Verklemmen des Druckelementes wird auch der kugelige Kopf der Knochenschraube in einer beliebigen Winkelstellung fixiert.

Die Nachteile dieser bekannten Vorrichtung sind die folgenden:
a) die Ausgestaltung des den Kugelkopf aufnehmenden hohlzylindrischen Druckelementes, welches lediglich zwei nur nach unten offene Längsschlitze aufweist, die sich auf die gleiche Seite hin erstrecken (in Richtung der Knochenschraube). Dadurch findet beim Klemmen kein homogenes Umschliessen des kugeligen Kopfes der Knochenschraube durch die Lamellen des geschlitzten Druckelementes statt. Dieses inhomogene Umschliessen des Kugelkopfes bewirkt eine reduzierte Klemmkraft;
b) die Knochenschraube muss vormontiert werden, indem sie zusammen mit dem Druckelement von oben durch die Ausnahme für das Spannelement eingeführt und in der Vorrichtung verdrehsicher befestigt wird, d.h. die Knochenschraube kann nicht unabhängig vom Aufnahmekopf eingedreht werden. Der Aufnahmekopf ermöglicht kein Aufsetzen nach der Implantation der Knochenschraube. Dadurch ergibt sich eine reduzierte Ubersicht beim Eindrehen der Knochenschraube mit aufgesetztem Aufnahmekopf. Wenn ein System mit unterschiedlich ausgeführten Aufnahmeköpfen angeboten werden soll, besteht das System auf Grund der designbedingten Vormontage der Knochenschraube aus n ∗ m Implantaten [wobei n = Anzahl der Kopfversionen und m = Anzahl der Schraubenlägen] anstatt bloss n + m Implantaten. Weiter nachteilig ist, dass kein situationsbedingtes Auswechseln der Aufnahmeköpfe in situ möglich ist.
c) die geringe Winkelstabilität, welche nur eine Zuggurtungsfixation gestattet;
d) mit dem Spannmittel werden gleichzeitig der Längsträger der Vorrichtung und der kugelförmige Kopf der Knochenschraube fixiert. Eine Lockerung des Spannmittels führt sofort zum Versagen des gesamten Fixationssystems; und
e) Dazu kommt, dass man je nach Situation die Verbindung zwischen der Vorrichtung und dem kugeligen Kopf der Knochenschraube unabhängig vom Längsträger fixieren möchte, um über den Längsträger ohne Winkelverlust zwischen Längsträger und Knochenschraube distrahieren, bzw. komprimieren zu können, was bei der bekannten Vorrichtung nicht möglich ist.

Hier will die Erfindung Abhilfe schaffen. Das Ziel der Erfindung liegt darin, eine Vorrichtung zur Verbindung eines Längsträgers mit einer Pedikelschraube mit folgenden Eigenschaften zu schaffen:
- einfache Handhabung
- Eindrehen des Schraubenteils der Vorrichtung auch ohne den Aufnahmekopf;
- mögliches nachträgliches Aufklicken des Aufnahmekopfes;
- je nach Situation und Vorliebe des Arztes freie Wahl von oder Mischung zwischen seitlich offenen, oben offenen oder geschlossenen Aufnahmeköpfen. z.B. nach oben offener Aufnahmekopf erleichtert das Einlegen des Längsträgers, währenddem eine seitliche Offnung seitliche Korrekturen ermöglicht.
- je nach Ausführung separate Fixation von Längsträger und kugeligem Kopf der Knochenschraube.

Die Erfindung löst die gestellte Aufgabe mit einer Vorrichtung, welche die Merkmale des Anspruchs 1 aufweist.

Die erfindungsgemässe Vorrichtung besteht aus einem den Längsträger aufnehmenden Aufnahmekopf, in welchen eine, einen vorzugsweise kugeligen Kopf aufweisende Pedikelschraube nachträglich eingeklickt und fixiert werden kann. Der Aufnahmekopf lässt sich in der gewünschten Anzahl und Variante einfach auf die bereits in den Wirbelkörpern implantierten Pedikelschrauben mit Kugelkopf aufklinken, so dass eine primäre Verbindung zwischen Längsträger und Pedikelschraube hergestellt wird. Durch das Einschrauben des Spannmittels in die Fixationsvorrichtung wird gleichzeitig der Längsträger in der Vorrichtung axial und relativ blockiert und die Vorrichtung winkelstabil fixiert. Dabei drückt die als Spannmittel dienende Stellschraube auf den in die Vorrichtung eingeführten Längsträger, dieser drückt auf einen alternierend geschlitzten Hohlkegelstumpf, welcher seinerseits aufgrund der konischen Ausbildung der Innenbohrung des Kopfes der Fixationsvorrichtung zusammengepresst wird und dadurch den vorzugsweise kugeligen Kopf der Pedikelschraube festklemmt. Für eine optimale Kraftübertragung vom Längsträger auf den Hohlkegelstumpf wird mit Vorteil ein steifes Zwischenelement zwischen Längsträger und Hohlkegelstumpf eingefügt. Je nach Gestaltung des Zwischenelementes und des Spannmittels ist es bei der vorliegenden Erfindung auch möglich, den Längsträger und den vorzugsweise kugeligen Kopf der Knochenschraube separat zu blockieren.
Die erfindungsgemässe Vorrichtung bietet somit gegenüber bekannten Vorrichtungen den Vorteil, dass die Pedikelschrauben nicht nur genau senkrecht zum Längsträger fixierbar sind, sondern eine Abwinkelung von bis zu ± 25° gestatten und auf Grund der Distanz zwischen Rotationszentrum des Aufnahmekopfes und der Achse des Längsträgers je nach dessen Dicke zwischen 4 und 10 mm zu einem gewissen Grad auch seitliche Abweichungen zwischen Längsträger und Pedikelschraube ausgeglichen werden können. Dies ist besonders wichtig, wenn der Längsträger ungenau angebogen wurde, was bei herkömmlichen Systemen zu grossen Schwierigkeiten bei der Montage führt. Ein weiterer Vorteil der erfindungsgemässen Vorrichtung besteht darin, dass je nach Ausführung des steifen Zwischenelementes und des Spannmittels, bzw. Ausführung der Vorrichtung Längsträger und der vorzugsweise kugelige Kopf der Knochenschraube separat blockiert werden können.

Eine bevorzugte Weiterbildung der erfindungsgemässen Vorrichtung besteht darin, dass der vorzugsweise kugelige Kopf der Pedikelschraube vorzugsweise mit einer Strukturierung in Form von Querrillen oder Querrippen versehen ist, um eine bessere Fixation (Verspannung gegen die Spannzange) zu erzielen.
Die Winkelstabilität kann noch dadurch erhöht werden, dass der Schraubenkopf aus einem relativ harten (z.B. Titan-Aluminium-Niob-Legierung) und der alternierend geschlitzte Hohlkegelstumpf aus einem relativ weichen Material (z.B. Titan in weichem Zustand) besteht.

Eine andere Möglichkeit zur Erhöhung der Winkelstabilität besteht darin, den vorzugsweise kugeligen Kopf der Pedikelschraube und/oder die komplementäre Kavität im Hohlkegelstumpf mit einer dreidimensionalen Strukturierung, z.B. in Form von Rillen aufzurauhen.

Dadurch wird die auf dem Schraubenkopf angebrachte Strukturierung aus relativ hartem Material in das relativ welche Material des Hohlkegelstumpf gedrückt.

Um die Pedikelschrauben in den Knochen eindrehen zu Können, sind sie im Kugelkopf vorzugsweise mit einem Innensechskant versehen. Wenn der aufklinkbare Kopf noch durchbohrt ist, kann wahlweise nur die Pedikelschraube oder gleich die gesamte Vorrichtung eingedreht werden. Letzteres hat vor allem den Vorteil, dass jederzeit die Vorrichtung weiter eingedreht oder zurückgedreht werden kann, um einen Höhenausgleich zu erreichen.

Der erfindungsgemäss verwendete Klickmechanismus hat noch den weiteren Vorteil gegenüber anderen, bekannten Klickmechanismen, z.B. gemäss der US-A-5 549 608, dass nach erfolgter Aufklickung, eine versehentliche Lösung der hergestellten Verbindung durch die vorzunehmenden Manipulationen nicht mehr möglich ist. Sollte die hergestellte Verbindung absichtlich wieder gelöst werden müssen, so ist dazu ein besonderes Instrument notwendig.

Die Erfindung und Weiterbildungen der Erfindung wird im folgenden anhand verschiedener Ausführungsbeispiele noch näher erläutert.

Es zeigen:
Fig. 1 eine perspektivische Explosionsdarstellung der erfindungsgemässen Vorrichtung mit einem oben offenen Aufnahmekopf zusammen mit einem Längsträger, einer Pedikelschraube mit Kugelkopf und einem Spannmittel;
Fig. 2 einen Längsschnitt durch die erfindungsgemässe Vorrichtung nach Fig. 1 längs der Zentralachse;
Fig. 3 eine perspektivische Ansicht einer modifizierten erfindungsgemässen Vorrichtung mit einem geschlossenen Kanal zur Aufnahme eines Längsträger, sowie einem Spannmittel.
Fig. 4 eine perspektivische Ansicht einer modifizierten erfindungsgemässen Vorrichtung mit einem seitlich (statt nach oben) geöffneten Kanal zur Aufnahme eines Längsträger, sowie einem Spannmittel;
Fig. 5 eine perspektivische Ansicht einer modifizierten erfindungsgemässen Vorrichtung mit einem seitlich geöffneten Kanal zur Aufnahme eines Längsträger, sowie einer zusätzlichen Hülse und Mutter zur Fixation des Längsträgers;
Fig. 6 eine perspektivische Ansicht einer modifizierten erfindungsgemässen Vorrichtung, bei welcher der Längsträger von der Zentralachse beabstandet ist;
Fig. 7 einen Längsschnitt durch eine modifizierte erfindungsgemässen Vorrichtung, bei welcher der Längsträger und der vorzugsweise kugelige Kopf der Knochenschraube separat blockiert werden können;
Fig. 8 einen Längsschnitt durch eine modifizierte erfindungsgemässe Vorrichtung mit Eigenschaften wie in Fig. 7 offenbart, mit dem Unterschied, dass die Stellschrauben für die Fixation des Längsträgers und des vorzugsweise kugeligen Kopfes der Knochenschraube mechanisch entkoppelt sind; und
Fig. 9 einen Längsschnitt durch eine modifizierte erfindungsgemässen Vorrichtung entsprechend zu Fig. 8 mit dem Vorteil, dass der Längsträger von oben einführbar ist.

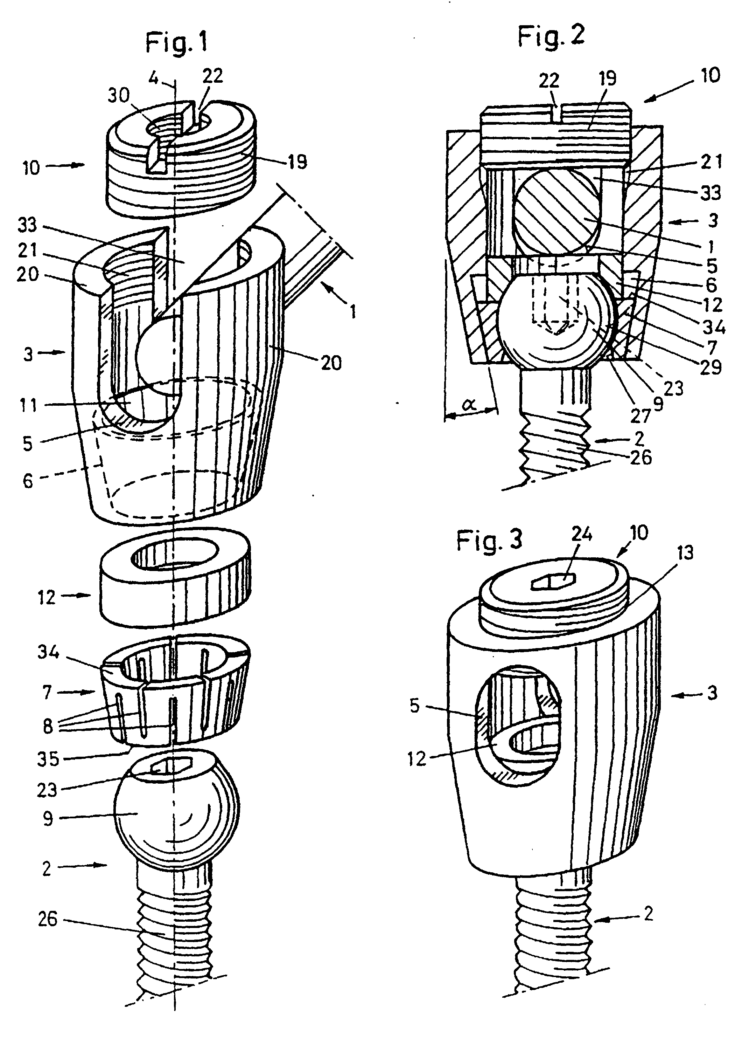

Die in Fig. 1 und 2 dargestellte erfindungsgemässe Vorrichtung besteht im wesentlichen aus einem Aufnahmekopf 3 mit der Zentralachse 4 und einem quer zur Zentralachse 4 verlaufenden, jochartigen, nach oben offenen Kanal 5 zur Aufnahme des Längsträgers 1, sowie einem nach unten geöffneten, sich konisch nach unten verjüngenden kegelstumpfförmigen Hohlraum 6 mit der Zentralachse 4, in welchem koaxial verschieblich eine hohlkegelstumpfförmige Spannzange 7 mit alternierenden Schlitzen 8 untergebracht ist, welche zur Aufnahme des Kopfes 9 einer Pedikelschraube 2 ausgebildet ist.

Wie in Fig. 2 dargestellt weist der kegelstumpfförmige Hohlraum 6 zweckmässigerweise einen halben Konuswinkel α/2 von ungefähr 6° auf. Die hohlkegelstumpfförmige Spannzange 7 mit alternierenden Schlitzen 8 weist einen entsprechenden halben Konuswinkel α/2 von ungefähr 6° auf. Die Spannzange 7 kann, dank ihrer Schlitze 8, im Durchmesser um etwa 0,4 mm gedehnt und bis zu 1 mm zusammengedrückt werden, so dass der Kopf 9 der Pedikelschraube 2 darin einklickbar ist.
Das Innere der Spannzange 7 ist zu diesem Zweck konkav, zweckmässigerweise hohlkugelförmig ausgebildet.

Der Hohlraum 6 reicht in axialer Richtung nach oben soweit in den Kanal 5 hinein, dass die Spannzange 7, bzw. das darauf liegende, vorzugsweise hohlzylindrische Zwischenelement 12 (z.B. in Form einer Unterlagsscheibe), durch den in den Kanal 5 eingeführten Längsträger 1 - wenn dieser mittels des Spannmittels 10 im Kanal 5 axial und rotativ fixiert wird - axial nach unten drückbar ist. Gleichzeitig mit der nach unten gerichteten axialen Verschiebung der Spannzange 7 im sich konisch nach unten verjüngenden, vorzugsweise kegelstumpfförmigen Hohlraum 6 erfolgt eine Verringerung des Durchmessers der Spannzange 7, so dass der zuvor in die Spannzange 7 von unten eingeklickte Kopf 9 der Pedikelschraube 2 festgeklemmt und in seiner relativen Lage zum Aufnahmekopf 3 (und damit zum Längsträger 1) fixiert wird. Dank der Entkoppelung zwischen Spannzange 7 und dem Zwischenelement 12 findet eine gleichmässige Umschliessung des Kopfes 9 statt, was zu einer optimalen Festigkeit der Verbindung zwischen Aufnahmekopf 3 und der Pedikelschraube 2 führt.

Das Spannmittel 10 weist ein Aussengewinde 19 auf und der Kanal 5 weist im Bereich der beiden jochartigen Schenkel 20 ein damit korrespondierendes Innengewinde 21 auf. Um die Stellschraube 19 in das Innengewinde 21 einzudrehen, weist sie an ihrem oberen Ende einen Querschlitz 22 und ein Innengewinde 30 auf, um ein entsprechend gestaltetes (zeichnerisch nicht dargestelltes) Manipulierinstrument aufnehmen zu können. An Stelle des Querschlitzes 22 und Innengewindes 30 kann zum Beispiel auch ein Innensechskant verwendet werden.

Die Funktion des Zwischenelementes 12 besteht in diesem speziellen Fall lediglich darin, eine möglichst gleichmässige Übertragung der Krätte vom Längsträger auf die Spannzange 7 zu ermöglichen, welche sich sonst im Hohlraum 6 leicht verkanten könnte.

Der Kopf 9 der Pedikelschraube 2 ist an seinem oberen Ende vorzugsweise mit einem Innensechskant 23 versehen, um die Pedikelschraube 2 mittels eines Sechskantschraubenziehers in den Knochen eindrehen zu können.

In Fig. 3 ist eine Variante der erfindungsgemässen Vorrichtung dargestellt, bei welcher der Aufnahmekopf 3 eine mit dem Kanal 5 kommunizierende, nach oben offene, gewindete Durchgangsbohrung 13 aufweist, in welche ein Spannmittel 10 mit Innensechskant 24 einschraubbar ist, derart dass ein im Kanal 5 eingeführter Längsträger 1 axial und rotativ fixierbar ist.

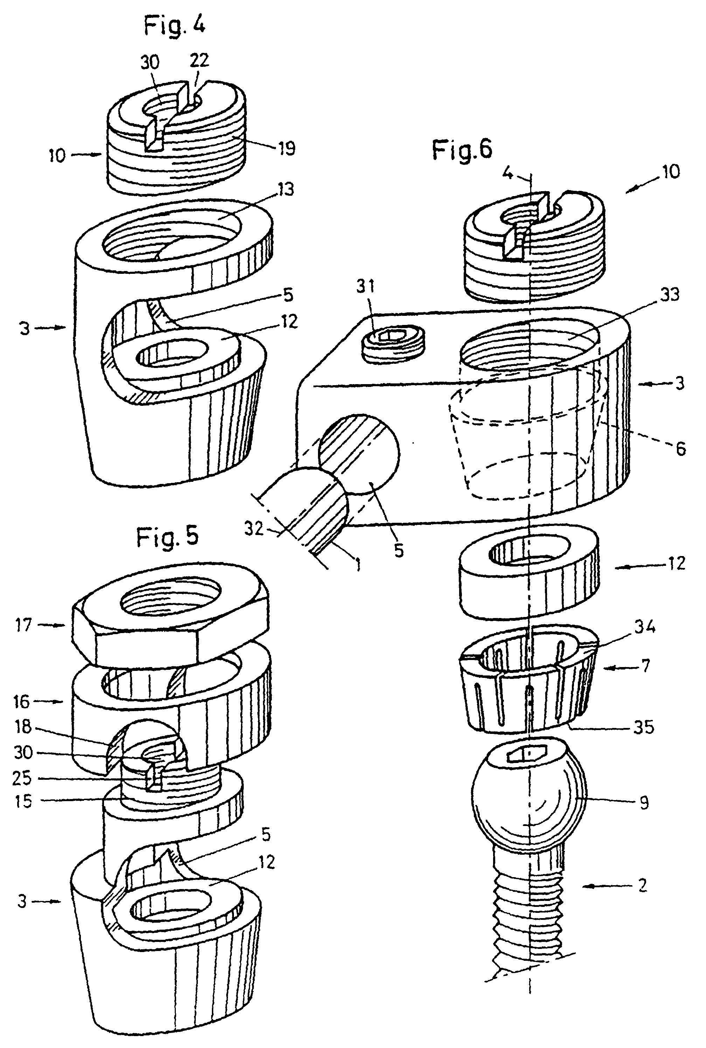

In Fig. 4 ist eine weitere Variante der erfindungsgemässen Vorrichtung nach Fig. 3 dargestellt, bei welcher der Kanal 5 nach der Seite offen ist, um den Längsträger 1 seitlich aufnehmen zu Können, was intraoperativ einige Vorteile bieten kann. Statt des Innensechskantes 24 kann das Spannmittel 10 auch einen Querschlitz 22 und ein Innengewinde 30 aufweisen. Der Querschlitz 22 und das Innengewinde 30 dienen in diesem Fall zur Aufnahme einer (zeichnerisch nicht dargestellten ) Manipulationsvorrichtung.

In Fig. 5 ist eine weitere Variante der erfindungsgemässen Vorrichtung dargestellt, bei welcher der Kanal 5 gleich wie bei der Variante nach Fig. 4 nach der Seite offen ist. Der Aufnahmekopf 3 weist jedoch an seinem oberen Abschnitt 14 ein Aussengewinde 15 mit Querschlitz 25 und Innengewinde 30 auf, über welches die hohlzylindrische Hülse 16 mit den Ausnehmungen 18 auf den Längsträger 1 gestülpt werden kann. Die Hülse 16 kann mit der, ein zum Aussengewinde 15 korrespondierendes Innengewinde aufweisenden Mutter 17 gegen den in den Kanal 5 eingeführten Längsträger 1 verspannt werden, so dass einerseits der Längsträger 1 axial und rotativ fixiert ist und anderseits auf das Zwischenelement 12, bzw. über die Öffnung 11 auf die Spannzange 7 drückt.

Bei der in Fig. 6 dargestellten weiteren Variante der erfindungsgemässen Vorrichtung ist der Aufnahmekopf 3 plattenförmig ausgebildet; er enthält neben dem sich konisch nach unten verjüngenden Hohlraum 6 mit der Zentralachse 4 den guer zu letzterer verlaufenden - rundum geschlossenen - Kanal 5 zur Aufnahme des Längsträger 1 mit der Längsachse 32. Die Fixation der Spannzange 7 erfolgt über das spannmittel 10, welche aut die Unterlagsscheibe 12 druckt. Die Unterlagsscheibe 12 kann bei dieser Ausführungsform ohne grosse Probleme auch weggelassen werden, so dass das Spannmittel direkt auf die Spannzange 7 drückt. Durch den Druck auf die Spannzange 7 gleitet diese innerhalb des sich konisch verjüngenden Hohlraums 6 nach unten und verengt sich dadurch im Durchmesser, so dass ein darin eingeführter, vorzugsweise kugeliger Kopf 9 einer Pedikelschraube 2 blockiert wird.
Die Fixation des Längsträger erfolgt bei dieser Ausführungsform separat durch eine zusätzliche Stellschraube 31, welche von oben in eine mit dem Kanal 5 kommunizierende Bohrung bis zur Anlage am Längsträger 1 eingeschraubt werden kann.

Der vorzugsweise kugelige Kopf 9 der Pedikelschraube 2 kann anstatt des Innensechskantes mit einer Vorrichtung versehen sein, um eine (zeichnerisch nicht dargestellte) Verlängerung aufsetzen zu können, die eine Manipulation der Pedikelschraube 2 durch das Spannmittel 10 (dies bedingt eine grosse, durchgehende Offnung) ermöglicht. Die Verbindung zwischen Pedikelschraube 2 und der Verlängerung ist vorzugsweise reversibel und rotations-, zug- und druckstabil.

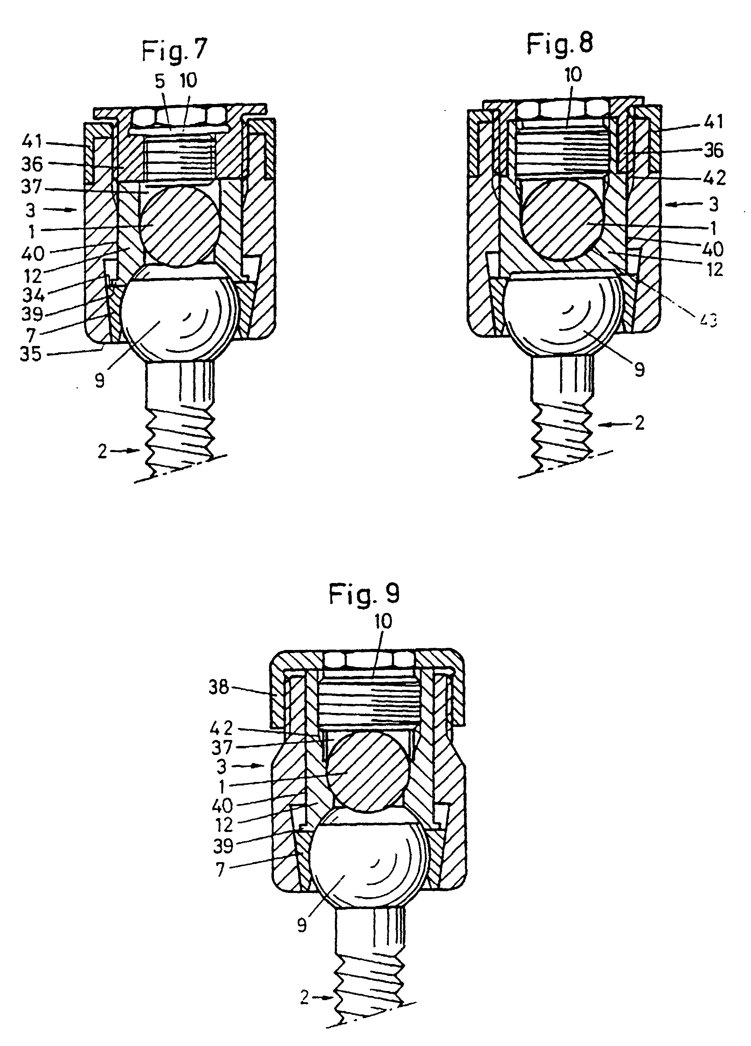

Die in Fig. 7 dargestellte Variante der erfindungsgemässen Vorrichtung zeigt die Anwendung von einem Spannmittel 10 und einer Spannschraube 36. Damit ist ein separates Fixieren von Längsträger 1 und Pedikelschraube 2 möglich. Die Spannschraube 36 wird im Aufnahmekopf eingeschraubt und fixiert nur den Kugelkopf 9 der Pedikelschraube 2. Die Schraube kann somit mit blockiertem Kugelkopf 9 entlang dem Längsträger verschoben werden, ohne den Winkel zwischen Längsträger 1 und Pedikelschraube 2 zu verlieren. Das Spannmittel 10 zur Fixierung des Längsträgers ist in der Spannschraube 36 integriert. Das Spannmittel 10 wird erst zur Blockierung des Längsträgers 1 angezogen, nachdem der Kugelkopf 9 durch die Spannschraube 36 fixiert wurde. Die Aufnahme des Längsträgers 1 im Aufnahmekopf 3 kann wie in Fig. 1 dargestellt durch einen nach oben offenen Kanal 5 erfolgen. Ebenso ist eine Aufnahme des Längsträgers 1 durch eine Bohrung wie in Fig. 3 dargestellt oder durch einen seitlich angebrachten, offenen Kanal 5 gemäss Fig. 4 möglich. Die Aufnahme des Längsträgers 1 im Zwischenelement 12 ist durch eine Bohrung 43 gemäss Fig. 8 möglich. Zur Vereinfachung der Montage ist bei einem oben offenen Aufnahmekopf 3 gemäss Fig. 1 ein mit einem oben offenen Kanal 37 versehenes Zwischenelement 12 gemäss Fig. 9 einsetzbar. Im Gegensatz zu Fig. 8 und 9 ist aber das Zwischenelement 12 nach oben so weit eröffnet, dass das Spannmittel 10 ungehindert direkt auf den Längsträger 1 drückt. Der Längsträger 1 kann dann von oben ins Zwischenelement 12 eingeführt werden. Das vorgängige Aufreihen des Zwischenelementes 12 auf dem Längsträger 1 bleibt aus. Ebenfalls ist es möglich, das Zwischenelement mit einem seitlichen Kanal 5 gemäss Fig. 4 zu versehen. Die Montage des Zwischenelementes 12 erfolgt vorzugsweise von der Seite der Spannzange 7 her. Ein Bord 39 am Zwischenelement 12 verhindert das Herausrutschen des Zwischenelementes 12 nach oben aus dem Aufnahmekopf 3. Das Herausrutschen nach unten wird durch das Spannelement 7 verhindert. Bei Verwenden eines wegen des Kanals 5 nach oben offenen Aufnahmekopfes 3 und einer Spannschraube 36 verhindert eine Sicherheitshülse 41 ein Aufweiten des Aufnahmekopfes beim Anziehen der Spannschraube 36. Anstelle der Spannschraube 36 ist auch eine Spannmutter 38 ähnlich wie Fig. 9 dargestellt möglich.

In Fig. 8 ist eine weitere Variante der erfindungsgemässen Vorrichtung dargestellt. Wie bei der in Fig. 7 dargestellten Variante fixiert eine zweite Spannschraube 36 den Kugelkopf 9 über das Zwischenelement 12, ohne den Längsträger festzuklemmen. Im Gegensatz zu der in Fig. 7 dargestellten Variante ist hier das Spannmittel 10, das zur Fixierung des Längsträgers 1 dient, im weiter hochgezogenen und mit einem Innengewinde 42 versehenen Zwischenelement 12 integriert. Das Zwischenelement 12 weist eine geschlossene, noch oben offene Gewindebohrung 42 auf. Der geschlossene Boden des Zwischenelementes 12 erlaubt es, homogen auf die Spannzange 7 zu drücken. Die Aufnahme des Längsträgers 1 im Aufnahmekopf 3 kann wie in Fig. 1 dargestellt durch einen nach oben offenen Kanal 5 erfolgen. Ebenso ist eine Aufnahme des Längsträgers 1 durch eine Bohrung wie in Fig. 3 dargestellt oder durch einen seitlich angebrachten, offenen Kanal 5 gemäss Fig. 4 möglich. Die Aufnahme des Längstragers 1 im Zwischenelement 12 ist durch eine Bohrung 43 möglich. Allerdings muss hier das Zwischenelement 12 auf den Längsträger 1 aufgereiht werden, bevor der Längsträger 1 in den Aufnahmekopf 3 eingebracht wird. Ein nach oben offener Kanal 5 am Zwischenelement 12 wie er in Fig. 7 dargestellt ist, ist auch möglich , wenn der Durchmesser des Längsträgers 1 etwa 3/5 des Kerndurchmessers des Innengewindes 42 beträgt. Bei Verwenden eines wegen des Kanals 5 nach oben offenen Aufnahmekopfes 3 und einer Spannschraube 36 verhindert eine Sicherheitshülse 41 ein Aufweiten des Aufnahmekopfes beim Anziehen der Spannschraube 36. Anstelle der Spannschraube 36 ist auch eine Spannmutter 38 ähnlich wie Fig. 9 dargestellt möglich.
Die in Fig. 9 dargestellte Variante der erfindungsgemässen Vorrichtung zeichnet sich, wie in Fig. 8 gezeigt, durch ein hochgezogenes Zwischenelement 12 und darin integriertem Spannmittel 10 zur Blockierung des Längsträgers aus. Hingegen wird die Spannzange 7 durch eine Spannmutter 38 blockiert. Diese Spannmutter 38 drückt auf das zwischenelement 12 und das Spannelement 7, wodurch der Kugelkopf 9 fixiert wird, ohne dass der Längsträger festgeklemmt wird. Ein zweites Spannmittel 10 läuft im Zwischenelement 12 und Klemmt den Längsträger 1 fest. Die Fixierungen von Kugelkopf 9 und Längsträger 1 sind so mechanisch entkoppelt. Die Aufnahme des Längsträgers im Aufnahmekopf 3 kann wie in Fig. 1 dargestellt durch einen nach oben offenen Kanal 5 erfolgen. Ebenso ist eine Aufnahme des Längsträgers 1 durch eine Bohrung wie in Fig. 3 dargestellt oder durch einen seitlich angebrachten, offenen Kanal 5 gemäss Fig. 4 möglich. Die Aufnahme des Längsträgers im Zwischenelement 12 ist durch eine Bohrung 43 gemäss Fig. 8 möglich. Zur Vereinfachung der Montage ist bei einem oben offenen Aufnahmekopf 3 gemäss Fig. 1 ein mit einem oben offenen Kanal 37 versehenes Zwischenelement 12 gemäss Fig. 9 einsetzbar. Der Längsträger 1 kann dann von oben ins Zwischenelement 12 eingeführt werden. Ein vorgangiges Aufreihen des Zwischenelementes 12 auf den Längsträger 1 kann ausbleiben. Ebenfalls ist es möglich, das Zwischenelement mit einem seitlichen Kanal 5 gemäss Fig. 4 zu versehen. Die Montage des Zwischenelementes 12 erfolgt vorzugsweise von der Seite der Spannzange 7 her. Ein Bord 39 am Zwischenelement 12 verhindert das Herausrutschen des Zwischenelementes 12 nach oben aus dem Aufnahmekopf. Das Herausrutschen nach unten wird durch das Spannelement 7 verhindert.

## Patentansprüche

1. Vorrichtung zur Verbindung eines Längsträger (1) mit einer Pedikelschraube (2) innerhalb eines Wirbelsäulenfixationssystems, mit einem Aufnahmekopf (3) mit der Zentralachse (4) und einem quer zur Zentralachse (4) verlaufenden Kanal (5) zur Aufnahme des Längsträgers (1), einer nach oben geöffneten Aufnahme (33) für ein Spannmittel (10;17) sowie einem nach unten geöffneten, sich konisch nach unten verjüngenden Hohlraum (6) mit der Zentralachse (4), in welchem koaxial gleitend eine Spannzange (7) mit der Zentralachse (4) untergebracht ist, welche zur federnden Aufnahme des Kopfes (9) einer Pedikelschraube (2) ausgebildet ist, wobei der Hohlraum (6) in axialer Richtung nach oben in die Aufnahme (33) des Spannmittels (10) mündet
**dadurch gekennzeichnet, dass**
die Spannzange (7) durch das Spannmittel (10;17) entweder direkt oder indirekt axial nach unten drückbar und radial zusammenpressbar ist, und dass
die Spannzange (7) hohlkegelstumpfförmig ausgebildet ist und mit mindestens vier axial verlaufenden Schlitzen (8) versehen ist, von denen eine Anzahl in die Basisfläche (34) und eine andere Anzahl in die Deckfläche (35) der hohlkegelstumpfförmigen Spannzange (7) münden.

2. Vorrichtung nach Anspruch 1, **dadurch gekennzeichnet, dass** die Schlitze (8) alternierend in die Basisfläche (34) und in die Deckfläche (35) der hohlkegelstumpfförmigen Spannzange (7) münden.

3. Vorrichtung nach Anspruch 1 oder 2, **dadurch gekennzeichnet, dass** die Spannzange (7) mit mindestens 6, vorzugsweise mindestens 8 Schlitzen (8) versehen ist.

4. Vorrichtung nach einm der Ansprüche 1 bis 3, **dadurch gekennzeichnet, dass** die Spannzange (7) rotationssymmetrisch zur Zentralachse (4) ausgebildet ist.

5. Vorrichtung nach einem der Ansprüche 1 bis 4, **dadurch gekennzeichnet, dass** die hohlkegelstumpfförmige Spannzange (7) einen halben Konuswinkel α/2 von 3° - 8°, vorzugsweise von 5° - 7° aufweist.

6. Vorrichtung nach einem der Ansprüche 1 bis 5, **dadurch gekennzeichnet, dass** der kegelstumpfförmige Hohlraum (6) einen halben Konuswinkel α/2 von 3° - 8°, vorzugsweise von 5° - 7° aufweist.

7. Vorrichtung nach einem der Ansprüche 1 bis 6, **dadurch gekennzeichnet, dass** die hohlkegelstumpfförmige Spannzange (7) im Durchmesser um 0,8 - 1,2 mm, vorzugsweise um 0,9 - 1,1 mm zusammendrückbar ist.

8. Vorrichtung nach einem der Ansprüche 1 bis 7, **dadurch gekennzeichnet, dass** die hohlkegelstumpfförmige Spannzange (7) im Durchmesser um 0,3 - 0,6 mm, vorzugsweise um 0,35 - 0,45 mm radial dehnbar ist.

9. Vorrichtung nach einem der Ansprüche 1 bis 8, **dadurch gekennzeichnet, dass** die hohlkegelstumpfförmige Spannzange (7) im Inneren konkav ausgebildet ist.

10. Vorrichtung nach Anspruch 9, **dadurch gekennzeichnet, dass** die hohlkegelstumpfförmige Spannzange (7) im Inneren hohlkugelschichtförmig ausgebildet ist.

11. Vorrichtung nach Anspruch 9, **dadurch gekennzeichnet, dass** die hohlkegelstumpfförmige Spannzange (7) im Inneren torusförmig ausgebildet ist.

12. Vorrichtung nach einem der Ansprüche 1 bis 8, **dadurch gekennzeichnet, dass** die hohlkegelstumpfförmige Spannzange (7) im Inneren hohlzylindrisch ausgebildet ist.

13. Vorrichtung nach einem der Ansprüche 1 bis 12, **dadurch gekennzeichnet, dass** die Spannzange (7) an ihrem unteren Abschnitt einen sich nach unten erweiternden Konus (27) aufweist, so dass der Schraubenschaft (26) der Pedikelschraube (2) in einem Winkel α von - 25° bis + 25° gegenüber der Zylinderachse (4) blockierbar ist.

14. Vorrichtung nach einem der Ansprüche 1 bis 13, **dadurch gekennzeichnet, dass** die Spannzange (7) in der Längsachse (4) durchgehend offen ist.

15. Vorrichtung nach einem der Ansprüche 1 bis 14, **dadurch gekennzeichnet, dass** sie zusätzlich eine Pedikelschraube (2) mit einem vorzugsweise kugeligen Kopf (9) umfasst.

16. Vorrichtung nach Anspruch 15, **dadurch gekennzeichnet, dass** der Kopf (9) der Pedikelschraube (2) mit einer Strukturierung vorzugsweise in Form von Querrillen, bzw. Querrippen (28) versehen ist.

17. Vorrichtung nach Anspruch 15 oder 16, **dadurch gekennzeichnet, dass** der Kopf (9) der Pedikelschraube (2) mit einem Innensechskant (16) versehen ist.

18. Vorrichtung nach einem der Ansprüche 1 bis 17, **dadurch gekennzeichnet, dass** das Innere der hohlkegelstumpfförmigen Spannzange (7) mit einer Strukturierung (29), vorzugsweise in Form von Querrillen, bzw. Querrippen versehen ist.

19. Vorrichtung nach einem der Ansprüche 15 bis 18, **dadurch gekennzeichnet, dass** die Pedikelschraube (9) aus einem härteren Material, vorzugsweise der Legierung Ti-Al-Nb, besteht als die Oberfläche im Inneren der hohlkegelstumpfförmigen Spannzange (7), welche vorzugsweise aus Reintitan besteht.

20. Vorrichtung nach einem der Ansprüche 15 bis 19, **dadurch gekennzeichnet, dass** der Durchmesser des kugeligen Kopfes (9) der Pedikelschraube (2) und der entsprechenden Ausnahme in der Spannzange (7) zwischen 6 und 10 mm, vorzugsweise zwischen 7,5 - 8,5 mm beträgt.

21. Vorrichtung nach einem der Ansprüche 15 bis 20, **dadurch gekennzeichnet, dass** der Abstand zwischen dem Zentrum des kugeligen Kopfes (9) und der Längsachse (32) des Längsträgers (1) zwischen 4 und 10 mm, vorzugsweise zwischen 6 - 8 mm beträgt.

22. Vorrichtung nach einem der Ansprüche 1 bis 21, **dadurch gekennzeichnet, dass** sie ein Spannmittel (10;17) umfasst, mit welchem der Längsträger (1) direkt oder indirekt, vorzugsweise über eine Hülse (16) axial und rotativ in lösbarer Weise im Aufnahmekopf (3) fixierbar ist.

23. Vorrichtung nach einem der Ansprüche 1 bis 22, **dadurch gekennzeichnet, dass** die Zentralachse (4) durch den Kanal (5) führt und die Verlängerung (11) des Hohlraumes (6) soweit in den Kanal (5) hineinreicht, dass das Spannmittel (10) die Spannzange (7) über den in den Kanal (5) eingeführten Längsträger (1) axial nach unten drücken und radial zusammenpressen kann.

24. Vorrichtung nach einem der Ansprüche 1 bis 23, **dadurch gekennzeichnet, dass** im Hohlraum (6) über der Spannzange (7) ein vorzugsweise hohlzylindrisches Zwischenelement (12) vorgesehen ist, auf welches der Längsträger (1) wirken kann, um die Spannzange (7) axial nach unten drücken zu können.

25. Vorrichtung nach einem der Ansprüche 1 bis 23, **dadurch gekennzeichnet, dass** in einem polygonalen Hohlraum (6) über der Spannzange (7) ein komplementär polygonales, vorzugsweise durchbohrtes Zwischenelement (12) vorgesehen ist, auf welches der Längsträger (1) wirken kann, um die Spannzange (7) axial nach unten drücken zu können.

26. Vorrichtung nach einem der Ansprüche 1 bis 25, **dadurch gekennzeichnet, dass** der Kanal (5) nach oben offen ist, derart dass der Aufnahmekopf (3) jochartig ausgebildet ist.

27. Vorrichtung nach einem der Ansprüche 1 bis 26, **dadurch gekennzeichnet, dass** der Aufnahmekopf (3) eine mit dem Kanal (5) kommunizierende, nach oben offene, gewindete Durchgangsbohrung (13) aufweist, in welche ein Spannmittel (10) einschraubbar ist, derart dass ein im Kanal (5) eingeführter Längsträger (1) axial und rotativ fixierbar ist.

28. Vorrichtung nach einem der Ansprüche 1 bis 25, **dadurch gekennzeichnet, dass** der Kanal (5) nach der Seite offen ist, um den Längsträger (1) seitlich aufnehmen zu können.

29. Vorrichtung nach einem der Ansprüche 1 bis 25, **dadurch gekennzeichnet, dass** der Kanal (5) nach der Seite offen ist, um den Längsträger (1) seitlich aufnehmen zu können und der Aufnahmekopf (3) an seinem oberen Abschnitt (14) ein Aussengewinde (15) aufweist und dass zusätzlich eine hohlzylindrische Hülse (16) mit Ausnehmungen (18) und eine zum Aussengewinde (15) korrespondierende Mutter (17) vorgesehen sind, womit ein im Kanal (5) eingeführter Längsträger (1) mittels der Hülse (16) axial und rotativ fixierbar ist.

30. Vorrichtung nach einem der Ansprüche 1 bis 21, **dadurch gekennzeichnet, dass** die Zentralachse (4) ausserhalb des Kanals (5) liegt.

31. Vorrichtung nach einem der Ansprüche 1 bis 29, **dadurch gekennzeichnet, dass** das Spannmittel (10) direkt auf die Spannzange (7) drückt.

32. Vorrichtung nach einem der Ansprüche 1 bis 29, **dadurch gekennzeichnet, dass** das Spannmittel (10) über ein vorzugsweise hohlzylindrisches Zwischenelement (12) indirekt auf die Spannzange (7) drückt.

33. Vorrichtung nach einem der Ansprüche 30 bis 32, **dadurch gekennzeichnet, dass** der Kanal (5) geschlossen ist.

34. Vorrichtung nach einem der Ansprüche 30 bis 32, **dadurch gekennzeichnet, dass** der Kanal (5) offen ist.

35. Vorrichtung nach einem der Ansprüche 30 bis 34, **dadurch gekennzeichnet, dass** das Spannelement für den Längsträger (1) eine Stellschraube (31) ist.

36. Vorrichtung nach einem der Ansprüche 30 bis 35, **dadurch gekennzeichnet, dass** der Abstand zwischen der Zentralachse (4) und der Längsachse (32) des Längsträgers (1) mindestens der halben Summe der Durchmesser des Längsträgers (1) und des Hohlraums (6) beträgt.

37. Vorrichtung nach einem der Ansprüche 1 bis 28, **dadurch gekennzeichnet, dass** das Spannelement über das Zwischenelement (12) auf die Spannzange (7) drückt und das Spannmittel (10) für den Längsträger (1) derart in der Spannschraube (36) integriert ist, dass Längsträger (1) und Kugelkopf (9) separat fixiert werden können.

38. Vorrichtung nach einem der Ansprüche 1 bis 28, **dadurch gekennzeichnet, dass** das Spannelement über das Zwischenelement (12) auf die Spannzange (7) drückt und das Spannmittel (10) für den Längsträger (1) in das Zwischenelement (12) integriert ist und somit Längsträger (1) und Kugelkopf (9) separat fixiert werden können.

39. Vorrichtung nach einem der Ansprüche 37 bis 38, **dadurch gekennzeichnet, dass** das Spannelement eine Spannschraube (36) ist.

40. Vorrichtung nach einem der Ansprüche 37 bis 38, **dadurch gekennzeichnet, dass** das Spannelement für die Spannzange (7) eine Spannmutter (38) ist.

## Claims

1. Device for the connection of a longitudinal support (1) with a pedicle screw (2) within a fixation system of the vertebral column, with a retainer head (3) with a central axis (4) and a channel (5) running transverse to the longitudinal axis (4) for the acceptance of the longitudinal support (1), with an upwardly open receiving means (33) for the acceptance of a tension means (10;17) as well as a downwardly open cavity (6) tapering downwards with the central axis (4) wherein a spring chuck (7) with the central axis (4) is accommodated coaxially gliding which is shaped to springably accept the head (9) of a pedicle screw (2) whereby the cavity (6) runs upwardly in axial direction into the receiving means (33) of the tension means (10;17),
**characterised in that**
the spring chuck (7) is downwardly pressable and radially compressible either directly or indirectly by means of the tension means (17;17); and that
the spring chuck (7) is shaped as a hollow truncated cone and provided with at least four slots (8) running axially whereby an amount is running into the basic area (34) and another amount is running into the top area (35) of the spring chuck (7) which is shaped as a hollow truncated cone.

2. Device according to claim 1, **characterized in that** the slots (8) are alternatively running into the basic area (34) and in the top area (35) of the spring chuck (7) which is shaped as a hollow truncated cone.

3. Device according to claim 1 or 2, **characterized in that** the spring chuck (7) is provided with at least 6, preferably at least 8 slots (8).

4. Device according to one of the claims 1 to 3, **characterized in that** the spring chuck (7) is shaped rotationally symmetrical to the central axis (4).

5. Device according to one of the claims 1 to 4, **characterized in that** the spring chuck (7) which is shaped as a hollow truncated cone provides a half cone angle α/2 of 3° - 8°, preferably of 5° - 7°.

6. Device according to one of the claims 1 to 5, **characterized in that** the cavity (6) which is shaped as a hollow truncated cone provides a half cone angle α/2 of 3° - 8°, preferably of 5° - 7°.

7. Device according to one of the claims 1 to 6, **characterized in that** the spring chuck (7) which is shaped as a hollow truncated cone is compressible in its diameter for 0,8 - 1,2 mm, preferably for 0,9 - 1,1 mm.

8. Device according to one of the claims 1 to 7, **characterized in that** the spring chuck (7) which is shaped as a hollow truncated cone is radially expandable in its diameter for 0,3 - 0,6 mm, preferably for 0,35 - 0,45 mm.

9. Device according to one of the claims 1 to 8, **characterized in that** the spring chuck (7) which is shaped as a hollow truncated cone is concavely shaped in its interior.

10. Device according to claim 9, **characterized in that** the spring chuck (7) which is shaped as a hollow truncated cone is shaped as a layer of a hollow sphere in its interior.

11. Device according to claim 9, **characterized in that** the spring chuck (7) which is shaped as a hollow truncated cone is toroidally shaped in its interior.

12. Device according to one of the claims 1 to 8, **characterized in that** the spring chuck (7) which is shaped as a hollow truncated cone is shaped as hollow cylinder in its interior.

13. Device according to one of the claims 1 to 12, **characterized in that** the spring chuck (7) provides in its lower section a cone (27) which extends downwardly so that the shaft (26) of the pedicle screw (2) is fixable at an angle α of - 25° to + 25° compared to the central axis (4).

14. Device according to one of the claims 1 to 13, **characterized in that** the spring chuck (7) is through open in direction of the central axis (4).

15. Device according to one of the claims 1 to 14, **characterized in that** it additionally comprises a pedicle screw (2) with a head (9) which is preferably spherical.

16. Device according to claim 15, **characterized in that** the head (9) of the pedicle screw (2) is provided with a structure preferably in the shape of transverse grooves respectively transverse fins.

17. Device according to claim 15 or 16, **characterized in that** the head (9) of the pedicle screw (2) is provided with a hexagon socket.

18. Device according to one of the claims 1 to 17, **characterized in that** the interior of the spring chuck (7) which is shaped as a hollow truncated cone is provided with a structure (29) preferably in the shape of transverse grooves respectively transverse fins.

19. Device according to one of the claims 15 to 18, **characterized in that** the pedicle screw (9) is made of a harder material, preferably of the alloy Ti-Al-Nb, than the surface of the interior of the spring chuck (7) which is shaped as a hollow truncated cone and is preferably made of technical pure titan.

20. Device according to one of the claims 15 to 19, **characterized in that** the diameter of the spherical head (9) of the pedicle screw (2) and of the corresponding hollow in the spring chuck (7) amounts to between 6 and 10 mm, preferably to between 7,5 - 8,5 mm.

21. Device according to one of the claims 15 to 20, **characterized in that** the distance between the center of the spherical head (9) and the longitudinal axis (32) of the longitudinal support (1) amounts to between 4 and 10 mm, preferably to between 6 - 8 mm.

22. Device according to one of the claims 1 to 21, **characterized in that** it comprises a tension means (10;17) through which the longitudinal support (1) is directly or indirectly, preferably via a sleeve (16) axial and rotatory fixable in the retainer head (3) in a detachable manner.

23. Device according to one of the claims 1 to 22, **characterized in that** the central axis (4) leads through the channel (5) and the extension (11) of the cavity (6) reaches into the channel (5) so far that the tension means (10) presses the spring chuck (7) downwards and radially compresses it via the longitudinal support (1) inserted in the channel (5).

24. Device according to one of the claims 1 to 23, **characterized in that** a preferably hollow cylindrical insert (12) is provided in the cavity (6) above the spring chuck (7) on that the longitudinal support (1) can be effective to press the spring chuck (7) axially downwards.

25. Device according to one of the claims 1 to 23, **characterized in that** in a polygonal cavity (6) above the spring chuck (7) a complementarily polygonal insert (12) is provided which is preferably penetrated on which the longitudinal support (1) can be effective to press the spring chuck (7) axially downwards.

26. Device according to one of the claims 1 to 25, **characterized in that** the channel (5) is upwardly open in such manner that the retainer head (3) is shaped as a yoke.

27. Device according to one of the claims 1 to 26, **characterized in that** the retainer head (3) provides a through hole (13) being threaded, upwardly open and communicating with the channel (5), wherein a tension means (10) is screwable such that a longitudinal support (1) inserted in the channel (5) is fixable axially and rotatively.

28. Device according to one of the claims 1 to 25, **characterized in that** the channel (5) is open to one side to sidewardly accept the longitudinal support (1).

29. Device according to one of the claims 1 to 25, **characterized in that** the channel (5) is open to one side to sidewardly accept the longitudinal support (1) and the retainer head (3) provides an external thread (15) at its upper section and that additionally a hollow cylindrical sleeve (16) with recesses (18) and a screw nut (17) corresponding to the external thread (15) are provided by what means a longitudinal support (1) inserted into the channel (5) is fixable axially and rotatively by means of the sleeve (16).

30. Device according to one of the claims 1 to 21, **characterized in that** the central axis (4) is situated outside of the channel (5).

31. Device according to one of the claims 1 to 29, **characterized in that** the tension means (10) directly presses on the spring chuck (7).

32. Device according to one of the claims 1 to 29, **characterized in that** the tension means (10) presses indirectly on the spring chuck (7) via an insert (12) which is preferably hollow cylindrical.

33. Device according to one of the claims 30 to 32, **characterized in that** the channel (5) is closed.

34. Device according to one of the claims 30 to 32, **characterized in that** the channel (5) is open.

35. Device according to one of the claims 30 to 34, **characterized in that** the tension means for the longitudinal support (1) is a locking screw (31).

36. Device according to one of the claims 30 to 35, **characterized in that** the distance between the central axis (4) and the longitudinal axis (32) of the longitudinal support (1) amounts at least half of the sum of the diameters of the longitudinal support (1) and the cavity (6).

37. Device according to one of the claims 1 to 28, **characterized in that** the tension means presses on the spring chuck (7) via the insert (12) and the tension means (10) for the longitudinal support (1) is integrated in the tensioning screw (36) such that the longitudinal support (1) and the spherical head (9) can be separately fixed.

38. Device according to one of the claims 1 to 28, **characterized in that** the tension means presses on the spring chuck (7) via the insert (12) and the tension means (10) for the longitudinal support (1) is integrated in the insert (12) thus that the longitudinal support (1) and the spherical head (9) can be separately fixed.

39. Device according to one of the claims 37 to 38, **characterized in that** the tension means is a tensioning screw (36).

40. Device according to one of the claims 37 to 38, **characterized in that** the tension means for spring chuck (7) is a tensioning nut (38).

## Revendications

1. Dispositif pour relier un support longitudinal (1) à une vis à pédicule (2) dans un système de fixation de la colonne vertébrale, présentant une tête de réception (3) avec un axe central (4) et un canal (5) qui s'étend transversalement par rapport à l'axe central (4), pour la réception du support longitudinal (1), un logement (33) ouvert par le haut pour un moyen de serrage (10 ; 17) ainsi qu'un espace creux (6) ouvert par le bas et qui se rétrécit coniquement vers le bas, d'axe central (4), dans lequel une pince de serrage (7) avec l'axe central (4) est installée à coulissement coaxial et est configurée pour la réception élastique de la tête (9) d'une vis à pédicule (2), l'espace creux (6) débouchant vers le haut dans la direction axiale dans le logement (33) du moyen de serrage (10), la pince de serrage (7) pouvant être repoussée axialement vers le bas et comprimée radialement, directement ou indirectement par le moyen de serrage (10 ; 17), et en ce que la pince de serrage (7) est configurée en forme de tronc de cône creux et est dotée d'au moins quatre fentes (8) qui s'étendent axialement parmi lesquelles un certain nombre débouchent dans la surface de base (34) et un autre nombre dans la surface de recouvrement (35) de la pince de serrage (7) en forme de tronc de cône creux.

2. Dispositif selon la revendication 1, **caractérisé en ce que** les fentes (8) débouchent de manière alternée dans la surface de base (34) et dans la surface de recouvrement (35) de la pince de serrage (7) en forme de tronc de cône creux.

3. Dispositif selon la revendication 1 ou 2, **caractérisé en ce que** la pince de serrage (7) est dotée d'au moins 6 et de préférence d'au moins 8 fentes (8).

4. Dispositif selon l'une des revendication 1 à 3, **caractérisé en ce que** la pince de serrage (7) présente une configuration à symétrie de rotation par rapport à l'axe central (4).

5. Dispositif selon l'une des revendications 1 à 4, **caractérisé en ce que** de la pince de serrage (7) en forme de tronc de cône creux présente un demi-angle de cône α/2 de 3° à 8°, de préférence de 5° à 7°.

6. Dispositif selon l'une des revendications 1 à 5, **caractérisé en ce que** l'espace creux (6) en forme de tronc de cône présente un demi-angle de cône α/2 de 3° à 8°, de préférence de 5° à 7°.

7. Dispositif selon l'une des revendications 1 à 6, **caractérisé en ce que** le diamètre de la pince de serrage (7) en forme de tronc de cône creux peut être comprimé de 0,8 à 1,2 mm, de préférence de 0,9 à 1,1 mm.

8. Dispositif selon l'une des revendications 1 à 7, **caractérisé en ce que** le diamètre de la pince de serrage (7) en forme de tronc de cône creux peut être allongée radialement de 0,3 à 0,6 mm, de préférence de 0,35 à 0,45 mm.

9. Dispositif selon l'une des revendications 1 à 8, **caractérisé en ce que** la pince de serrage (7) en forme de tronc de cône creux présente à l'intérieur une configuration concave.

10. Dispositif selon la revendication 9, **caractérisé en ce que** l'intérieur de la pince de serrage (7) est configuré en forme de sphère creuse.

11. Dispositif selon la revendication 9, **caractérisé en ce que** l'intérieur de la pince de serrage (7) en forme de tronc de cône creux présente la configuration d'un tore.

12. Dispositif selon l'une des revendications 1 à 8, **caractérisé en ce que** l'intérieur de la pince de serrage (7) en forme de tronc de cône creux est configurée en forme de cylindre creux.

13. Dispositif selon l'une des revendications 1 à 12, **caractérisé en ce que** sur sa partie inférieure, la pince de serrage (7) présente un cône (27) qui s'élargit vers le bas de telle sorte que la tige filetée (26) de la vis à pédicule (2) puisse être bloquée sous un angle α de -25° à +25° par rapport à l'axe (4) du cylindre.

14. Dispositif selon l'une des revendications 1 à 13, **caractérisé en ce que** la pince de serrage (7) est ouverte en continu le long de son axe longitudinal (4).

15. Dispositif selon l'une des revendications 1 à 4, **caractérisé en ce qu'**il comporte en supplément une vis à pédicule (2) dotée d'une tête (9) de préférence sphérique.

16. Dispositif selon la revendication 15, **caractérisé en ce que** la tête (9) de la vis à pédicule (2) est dotée d'une structuration qui présente de préférence la forme de rainures transversales ou de nervures transversales (28).

17. Dispositif selon la revendication 15 ou 16, **caractérisé en ce que** la tête (9) de la vis à pédicule (2) est dotée d'un creux à six pans (16).

18. Dispositif selon l'une des revendications 1 à 17, **caractérisé en ce que** l'intérieur de la pince de serrage (7) en forme de tronc de cône creux est doté d'une structuration (29) qui présente de préférence la forme de rainures transversales ou de nervures transversales.

19. Dispositif selon l'une des revendications 15 à 18, **caractérisé en ce que** la vis à pédicule (9) est constituée d'un matériau, de préférence un alliage de Ti-Al-Nb, plus dur que la surface de l'intérieur de la pince de serrage (7) en forme de tronc de cône creux qui est de préférence constitué de titane pur.

20. Dispositif selon l'une des revendications 15 à 19, **caractérisé en ce que** le diamètre de la tête sphérique (9) de la vis à pédicule (2) et du logement correspondant dans la pince de serrage (7) est compris entre 6 et 10 mm, de préférence entre 7,5 et 8,5 mm.

21. Dispositif selon l'une des revendications 15 à 20, **caractérisé en ce que** la distance entre le centre de la tête sphérique (9) et l'axe longitudinal (32) du support longitudinal (1) est compris entre 4 et 10 mm, de préférence entre 6 et 8 mm.

22. Dispositif selon l'une des revendications 1 à 21, **caractérisé en ce qu'**il comprend un moyen de serrage (10 ; 17) par lequel le support longitudinal (1) peut être fixé dans la tête de réception (3) directement ou indirectement, de préférence par l'intermédiaire d'une douille (36), dans le sens axial et en rotation et de manière libérable.

23. Dispositif selon l'une des revendications 1 à 22, **caractérisé en ce que** l'axe central (4) passe par le canal (5) et le prolongement (11) de l'espace creux (6) pour pénétrer dans le canal (5) de telle sorte que le moyen de serrage (10) puisse repousser axialement vers le bas et comprimer radialement la pince de serrage (7) par l'intermédiaire du canal longitudinal (1) inséré dans le canal (5).

24. Dispositif selon l'une des revendications 1 à 23, **caractérisé en ce qu'**un élément intermédiaire (12) de préférence cylindrique creux, sur lequel le support longitudinal (1) peut agir pour repousser la pince de serrage (7) axialement vers le bas est prévu dans l'espace creux (6) au-dessus de la pince de serrage (7).

25. Dispositif selon l'une des revendications 1 à 23, **caractérisé en ce qu'**un élément intermédiaire (12) polygonal, de préférence traversé par un alésage et sur lequel le support longitudinal (1) peut agir pour repousser la pince de serrage (7) axialement vers le bas est prévu dans un espace polygonal creux complémentaire (6) situé au-dessus de la pince de serrage (7).

26. Dispositif selon l'une des revendications 1 à 25, **caractérisé en ce que** le canal (5) est ouvert par le haut de telle sorte que la tête de réception (3) est configuré en forme d'étrier.

27. Dispositif selon l'une des revendications 1 à 26, **caractérisé en ce que** la tête de réception (3) présente un alésage de passage (13) fileté, ouvert par le haut, en communication avec le canal (5) et dans lequel un moyen de serrage (18) peut être vissé de manière à pouvoir fixer axialement et en rotation un support longitudinal (1) inséré dans le canal (5).

28. Dispositif selon l'une des revendications 1 à 25, **caractérisé en ce que** le canal (5) est ouvert sur le côté pour pouvoir recevoir le support longitudinal (1) par le côté.

29. Dispositif selon l'une des revendications 1 à 25, **caractérisé en ce que** le canal (5) est ouvert sur le côté pour pouvoir recevoir le support longitudinal (1) par le côté, la tête de réception (3) présentant un filet extérieur (15) dans sa partie supérieure (14), et **en ce qu'**en plus, une douille cylindrique creuse (16) dotée de découpes (18) et un écrou (17) qui correspond au filet extérieur (15) sont prévus pour pouvoir fixer axialement et en rotation au moyen de la douille (16) un support longitudinal (1) inséré dans le canal (5).

30. Dispositif selon l'une des revendications 1 à 21, **caractérisé en ce que** l'axe central (4) est situé à l'extérieur du canal (5).

31. Dispositif selon l'une des revendications 1 à 29, **caractérisé en ce que** le moyen de serrage (10) repousse directement la pince de serrage (7).

32. Dispositif selon l'une des revendications 1 à 29, **caractérisé en ce que** le moyen de serrage (10) repousse indirectement la pince de serrage (7) par l'intermédiaire d'un élément intermédiaire (12) de préférence cylindrique creux.

33. Dispositif selon l'une des revendications 30 à 32, **caractérisé en ce que** le canal (5) est fermé.

34. Dispositif selon l'une des revendications 30 à 32, **caractérisé en ce que** le canal (5) est ouvert.

35. Dispositif selon l'une des revendications 30 à 34, **caractérisé en ce que** l'élément de serrage du support longitudinal (1) est une vis de serrage (31).

36. Dispositif selon l'une des revendications 30 à 35, **caractérisé en ce que** la distance entre l'axe central (4) et l'axe longitudinal (32) du support longitudinal (1) vaut au moins la demi-somme des diamètres du support longitudinal (1) et de l'espace creux (6).

37. Dispositif selon l'une des revendications 1 à 28, **caractérisé en ce que** l'élément de serrage repousse la pince de serrage (7) par l'intermédiaire de l'élément intermédiaire (12) et **en ce que** le moyen de serrage (10) du support longitudinal (1) est intégré dans la vis de serrage (36) de telle sorte que le support longitudinal (1) et la tête sphérique (9) puissent être fixés séparément.

38. Dispositif selon l'une des revendications 1 à 28, **caractérisé en ce que** l'élément de serrage repousse la pince de serrage (7) par l'intermédiaire de l'élément intermédiaire (12) et **en ce que** l'élément de serrage (10) du support longitudinal (1) est intégré dans l'élément intermédiaire (12), le support longitudinal (1) et la tête sphérique (9) pouvant être ainsi fixés séparément.

39. Dispositif selon l'une des revendications 37 à 38, **caractérisé en ce que** l'élément de serrage est une vis de serrage (36).

40. Dispositif selon l'une des revendications 37 à 38, **caractérisé en ce que** l'élément de serrage de la pince de serrage (7) est un écrou de serrage (38).
